# EUROPÄISCHE PATENTSCHRIFT

(11) **EP 1 511 721 B1**
(45) Veröffentlichungstag und Bekanntmachung des Hinweises auf die Patenterteilung: **01.07.2009**
(21) Anmeldenummer: 03735558.3
(22) Anmeldetag: 05.06.2003
(51) Int. Cl.: C07C 279/18, A61K 31/155, A61P 35/00

(54) **GUANIDINOPHENYLALANIN-VERBINDUNGEN ALS UROKINASE-INHIBITOREN**
GUANIDINO PHENYLALANIN COMPOUNDS USED AS UROKINASE INHIBITORS
COMPOSES GUANIDINO-PHENYLALANINE EN TANT QU'INHIBITEURS DE L'UROKINASE

(30) Priorität: 11.06.2002 DE 10225876
(43) Veröffentlichungstag der Anmeldung: 09.03.2005
(73) Patentinhaber: Wilex AG, 81675 München (DE)
(72) Erfinder: SPERL, Stefan, 81549 München (DE)
(74) Vertreter: Weiss, Wolfgang
(86) Internationale Anmeldenummer: PCT/EP2003/005918
(87) Internationale Veröffentlichungsnummer: WO 2003/103644

(56) Entgegenhaltungen:
- WO-A-00/04954
- WO-A-01/70204
- WO-A-02/074756
- DE-A- 10 029 014
- DE-A- 19 940 389
- STURZEBECHER J ET AL: "3-Amidinophenylalanine-based Inhibitors of Urokinase" BIOORGANIC & MEDICINAL CHEMISTRY LETTERS, OXFORD, GB, Bd. 9, Nr. 21, 1. November 1999 (1999-11-01), Seiten 3147-3152, XP004181024 ISSN: 0960-894X
- MAGDOLEN V ET AL: "Natural and synthetic inhibitors of the tumor-associated serine protease urokinase-type plasminogen activator." ADVANCES IN EXPERIMENTAL MEDICINE AND BIOLOGY. UNITED STATES 2000, Bd. 477, 2000, Seiten 331-341, XP008021329 ISSN: 0065-2598
- HEECHUNG YANG ET AL: "SELECTIVE INHIBITION OF UROKINASE BY SUBSTITUTED PHENYLGUANIDINES: QUANTITATIVE STRUCTURE-ACTIVITY RELATIONSHIP ANALYSES" JOURNAL OF MEDICINAL CHEMISTRY, AMERICAN CHEMICAL SOCIETY. WASHINGTON, US, Bd. 33, Nr. 11, 1990, Seiten 2956-2961, XP002059264 ISSN: 0022-2623
- SPERL S ET AL: "(4-Aminomethyl)phenylguanidine derivatives as nonpeptidic highly selective inhibitors of human urokinase" PROCEEDINGS OF THE NATIONAL ACADEMY OF SCIENCES OF USA, NATIONAL ACADEMY OF SCIENCE. WASHINGTON, US, Bd. 97, Nr. 10, 9. Mai 2000 (2000-05-09), Seiten 5113-5118, XP002169711 ISSN: 0027-8424
- NIENABER VICKI ET AL: "Re-engineering of human urokinase provides a system for structure-based drug design at high resolution and reveals a novel structural subsite." JOURNAL OF BIOLOGICAL CHEMISTRY, Bd. 275, Nr. 10, 10. März 2000 (2000-03-10), Seiten 7239-7248, XP002253397 ISSN: 0021-9258
- IROLA K. ET AL: 'Urokinase plasminogen activator is expressed by basal keratinocytes before interstitial collagenase, stromelysin-1, and laminin-5 in experimentally induced dermatitis herpetiformis lesions' JOURNAL OF INVESTIGATIVE DERMATOLOGY Bd. 108, 1997, Seiten 7 - 11
- FRAKI J.E. ET AL: 'Correlation of epidermal plasminogen activator activity with disease activity in psoriasis' BRITISH JOURNAL OF DERMATOLOGY Bd. 108, 1983, Seiten 39 - 44
- GRANT M.B.; GUAY C.: 'Plasminogen activator production by human retinal endothelial cells of nondiabetic and diabetic origin' INVESTIGATIVE OPHTHALMOLOGY & VISUAL SCIENCE Bd. 32, Nr. 1, Januar 1991, Seiten 53 - 64
- KANSE S.M. ET AL: 'Induction of vascular SMC proliferation by urokinase indicates a novel mechanism of action in vasoproliferative disorders' ARTERIOSCLEROSIS, THROMBOSIS, AND VASCULAR BIOLOGY Bd. 17, 1997, Seiten 2848 - 2854
- MAY A.E. ET AL: 'Urokinase receptor (CD87) regulates leukocyte recruitement via beta-2 integrins in vivo' THE JOURNAL OF EXPERIMENTAL MEDICINE Bd. 188, Nr. 6, 21 September 1998, Seiten 1029 - 1037
- MULLINS D.E.; ROHRLICH S.T.: 'The role of proteinases in cellular invasiveness' BIOCHIMICA ET BIOPHYSICA ACTA Bd. 695, 1983, Seiten 177 - 214
- PREISSNER K.T. ET AL: 'Molecular crosstalk between adhesion receptors and proteolytic cascades in vascular remodelling' THROMBOSIS AND HAEMOSTASIS Bd. 78, Nr. 1, 1997, Seiten 88 - 95
- SCHAFER B.M. ET AL: 'Plasminogen activation in bullous pemphigoid immunohistology reveals urokinase type plasminogen activator, its receptor and plasminogen activator inhibitor type-2 in lesional epidermis' AUTOIMMUNITY Bd. 23, 1996, Seiten 155 - 164
- WILINSON J.E. ET AL: 'Role of plasminogen activator in pemphigus vulgaris' AMERICAN JOURNAL OF PATHOLOGY Bd. 134, Nr. 3, März 1989, Seiten 561 - 569

## Beschreibung

Die Erfindung betrifft die Verwendung von Derivaten des 3-Guanidinophenylalanins als Urokinase-Inhibitoren, insbesondere zur Behandlung von malignen Tumoren und der Metastasierung bzw. als Mittel zum Targeting von Lymphzellen, und zur Behandlung von Erkrankungen des Lymphgewebes, insbesondere von Lymphomen.

Die Fähigkeit solider Tumoren zur Ausbreitung und Metastasierung in umgebendes Gewebe korreliert mit dem Abbau bzw. Umbau der extrazellulären Matrix (Tumorstroma) in der Umgebung der Tumorzelle bzw. mit deren Fähigkeit zur Durchdringung der Basalmembran. Obwohl die (patho)biochemischen Zusammenhänge noch nicht endgültig aufgeklärt sind, kommen dem Plasminogenaktivator Urokinase (uPA) und dem Urokinaserezeptor (uPAR) eine zentrale Bedeutung zu. uPA vermittelt die proteolytische Spaltung von Plasminogen zu Plasmin. Plasmin wiederum ist eine Protease mit breitem Wirkspektrum, die Komponenten der extrazellulären Matrix wie Fibrin, Fibronektin, Laminin und das Proteingerüst der Proteoglykane direkt abzubauen vermag. Außerdem kann Plasmin "latente" Metalloproteasen und das inaktive Proenzym von uPA, pro-uPA, aktivieren.

Tumorzellen und nichtmaligne Zellen des Tumorstromas synthetisieren und sezernieren das enzymatisch inaktive Proenzym pro-uPA. Proteasen, wie z.B. Plasmin oder die Kathepsine B und L, spalten pro-uPA durch limitierte Proteolyse zur aktiven Serinprotease HMW-uPA (HMW =high molecular weight). pro-uPA und die aktive Protease HMW-uPA binden an den Zelloberflächenrezeptor uPAR (CD87). Plasmin(ogen) bindet ebenfalls an spezifische Rezeptoren auf der Plasmamembran der Tumorzelle, wodurch eine Fokussierung und Amplifikation der Plasminogenaktivierung in der direkten Umgebung der Tumorzelle erreicht wird. Invasiven Zellen ist somit die Möglichkeit gegeben, die extrazelluläre Matrix abzubauen, ohne sich der für eine gerichtete Bewegung notwendigen Unterlagen durch Proteolyse zu entziehen.

In verschiedenen zellbiologischen Studien konnte gezeigt werden, dass dem zellassoziiertenPlasminogenaktivator-Systeminnerhalbderkaskadenartigen Reaktionswege tumorassoziierter Proteolysesyteme ein besonderer Stellenwert zukommt (Wilhelm et al. (1994) The Urokinase/Urokinase receptor system: A new target for cancer therapy? In: Schmitt M., Graeff H., Kindermann G. (Hrsg): Prospects in Diagnosis and Treatment of Cancer. International Congress Series, Excerpta Medica 1050, Amsterdam, Elsevier 1994, pp145-156). An Kulturen humaner Kolonkarzinomzellen wurde beobachtet, dass deren Fähigkeit, eine extrazelluläre Matrix zu durchwandern, vom Sättigungsgrad der uPA-Rezeptoren mit aktivem uPA abhängig ist (Hollas et al., Cancer Res. 51 (1991), 3690-3695). Ebenfalls im Zellkulturmodell wurde eine Reduktion des invasiven Potenzials von Zellen beobachtet, wenn die proteolytische Aktivität von uPA durch PAI-1 (Cajot et al., Proc. Natl. Acad. Sci. USA 87 (1990), 6939-6943) oder PAI-2 (Baker et al., Cancer Res. 50 (1990), 4676-4684) gehemmt wurde. Ein vergleichbarer Effekt wurde bei Hemmung der Bindung von uPA an die Zelloberfläche durch Blockierung des Rezeptors mittels proteolytisch inaktiver uPA-Varianten erzielt (Cohen et al., Blood 78 (1991), 479-487; Kobayashi et al., Br. J. Cancer 67 (1993), 537-544). Auch die Transfektion epidermoider Karzinomzellen mit einem Plasmid, das ein Antisense-Transkript gegen einen Teil von uPAR exprimiert, führte durch Unterdrückung der uPAR-Synthese zur Verringerung der Invasivität dieser Zellen (Kook, EMBO J. 13 (1994), 3983-3991). Gegen uPA und PAI-1 gerichtete Antikörper reduzierten das invasive Potential von Lungenkrebszellen *in vitro* (Liu et al., Int. J. Cancer 60 (1995), 501-506). Der Einfluss des Plasminogenaktivator-Systems auf den Metastasierungsprozess konnte auch in Tumor-Tiermodellen belegt werden. So wurden die durch menschliche Karzinomzellen verursachte Bildung von Lungenmetastasen in Hühnerembryos durch Zugabe von Antikörpern gegen uPA fast vollständig verhindert (Ossowski und Reich, Cell 35 (1983), 61 1-619). Metastasierende menschliche Karzinomzellen wurden mit einem Expressionsplasmid transfiziert, das für eine proteolytisch inaktive, aber uPAR-bindende uPA-Mutante kodierte. Im Mausmodell zeigte sich, dass die Karzinomzellen, die inaktives uPA synthetisierten, nach Injektion im Vergleich zu den nichttransfizierten Zellen eine signifikant geringere Anzahl an Metastasen bildeten (Crowley et al., Proc. Natl. Acad. Sci. USA 90 (1993), 5021-5025). Nach Verabreichung von uPA-Antisense Oligonukleotiden wurde darüber hinaus eine Inhibierung der intraperitonealen Ausbreitung von humanen Ovarialkarzinomzellen in Nacktmäusen beobachtet (Wilhelm et al., Clin. Exp. Metast. 13 (1995), 296-302).

In den letzten Jahren wurde die klinische Relevanz von Faktoren des Plasminogenaktivator-Systems (uPA, uPAR, PAI-1 und PAI-2) für die Prognose von Patienten mit soliden malignen Tumoren intensiv untersucht. Dabei erwies sich der uPA-Antigengehalt bei verschiedenen Tumoren (z.B. Brust, Eierstock, Magen, Lunge, Niere etc.) sowohl für das rezidivfreie Überleben als auch für das Versterben als ein starker Prognosefaktor (siehe beispielsweise Schmitt et al., J. Obstet. Gynaecol. 21 (1995), 151-165; Jaenicke et al., Breast Cancer Res. Treat. 24 (1993), 195-208; Kuhn et al., Gynecol. Oncol. 55 (1994), 401-409; Nekarda et al., Lancet 343 (1994), 117; Pedersen et al., Cancer Res. 54 (1994), 4671-4675). Ebenso korrelieren erhöhte Konzentrationen an uPAR in Lungen- (Pedersen et al., supra) und Brustkrebsgewebe (Duggan et al., Int. J. Cancer 61 (1995), 597-600; Ronne et al., Breast Cancer Res. Treat. 33 (1995), 199-207) sowie bei Magenkrebs, sowohl im Tumorgewebe selbst (Heiss et al., J. Clin. Oncol. 13 (1995), 2084-2093) als auch bei den ins Knochenmark ausgestreuten Tumorzellen (Heiss et al., Nature Medicine 1 (1995), 1035-1039) mit einer schlechten Prognose.

Der Einsatz von sythetischen uPA-Inhibitoren bietet die Möglichkeit, die Invasion und Ausbreitung von Tumorzellen zu unterdrücken. Allerdings ist die Entwicklung spezifischer uPA-Inhibitoren mit Schwierigkeiten behaftet, da der Plasminogenaktivator vom Gewebetyp (tPA) eine identische Spezifität für die Spaltung der Peptidbindung Arg560/Val561 von Plasminogen aufweist. In den meisten Fällen hemmen daher niedermolekulare uPA-Inhibitoren auch tPA und damit auch tPA-vermittelte Fibrinolyse. Außerdem muss gewährleistet sein, dass synthetische uPA-Inhibitoren keine starke Hemmung von Plasmin zeigen.

Trotz dieser Einschränkungen sind einige Hemmstoffe bekannt, die eine gewisse Spezifität gegenüber uPA, jedoch geringe inhibitorische Kapazität besitzen, wie etwa Benzamidin- und β-Naphthamidin-Derivate, wobei die wirksamste Verbindung uPA mit Kᵢ=2,2 µmol/l hemmt (Stürzebecher und Markwardt, Pharmazie 33 (1978), 599), oder Amilorid mit einem Kᵢ = 7 µmol/l (Vassalli und Belin, FEBS. Lett. 214 (1987), 187-191).

DE-A-30 35 086 offenbart Cyclohexancarbonsäurederivate, die inhibitorische Wirkungen auf Proteasen wie Trypsin, Chymotrypsin, Thrombin oder uPA, haben. Die untersuchten Verbindungen zeigen jedoch nur eine recht geringe und darüber hinaus unspezifische uPA-Inhibierung. EP-A-0 183 271 offenbart Lysinderivate und deren Verwendung als Proteaseinhibitoren. Es wird auch ein Benzamidino-Lysinderivat (Verbindung 108) beschrieben, das *in vitro* eine uPA-Hemmung, jedoch auch eine vergleichbare Wirkung auf andere Proteasen wie Trypsin oder Plasma-Kallikrein aufweist. WO 95/17885 offenbart niedermolekulare Polypeptide als uPA-Inhibitoren.

Eine weitere Klasse von bekannten uPA-Inhibitoren stellen 4-substituierte Benzothiophen-2-carboxamidine mit einem Kᵢ =0,16 mmol/l im Falle von Benzothiophen-623 dar (Towle et al., Cancer Res. 53 (1993), 2553-2559). Diese Hemmstoffe haben eine signifikant höhere Affinität für uPA im Vergleich zu tPA und Plasmin. Auch uPAR-gebundenes uPA wird mit hoher Effizienz gehemmt. Ein Nachteil dieser Substanzen liegt allerdings darin, dass die chemische Synthese kompliziert ist und kaum Möglichkeiten für die Modifizierung der Struktur vorhanden sind bzw. bisher gezeigt werden konnte.

Nα-Arylsulfonyl- und Nα-Arylsulfonyl-amino-acyl-Derivate des 3-Amidinophenylalanins sind als selektive Hemmstoffe von Thrombin (Markwardt et al., Thromb. Res. 17 (1980), 425-431) bzw. von Gerinnungsfaktor Xa (Stürzebecher et al., Thromb. Res. 54 (1989), 245-252) bekannt. Auch in WO 92/08709 und WO 96/05189 wird die Verwendung von Amidinophenylalaninderivaten als Hemmstoffe für die Blutgerinnung, insbesondere als Hemmstoffe für Thrombin, offenbart. In WO 94/18185 werden Amidino- und Guanidinoderivate des Phenylalanins und deren Verwendung als Hemmstoffe der Blutgerinnung, insbesondere als antithrombotisch wirkende Stoffe offenbart.

Stürzebecher et al. (Bioorganic & Medicinal Chemistry Letters 1999, 9:3147-3152) offenbaren die Synthese und anti-uPA-Aktivität von 3-Amidinophenylalaninamiden.

Magdolen et al. (in Cellular Peptidases in Immune Functions and Diseases 2, Hrsg. Langner und Ansorge, Kluwer Academic/Plenum Publishers 2000, Seiten 331-341) offenbaren, dass monosubstituierte Phenylguanidine eine geringe oder keine Selektivität für uPa aufweisen. Intensiv wurden Piperidide und Piperazide des 3-Amidinophenylalanins untersucht, unter denen auch Leitstrukturen zur Hemmung fibrinolytischer Enzyme gefunden wurden (Stürzebecher et al., J. Enzyme Inhibition 9,87-99, 1995; Stürzebecher et al., J. Med. Chem. 40, 3091-3099, 1997). Während bei Stürzebecher et al. (1995) nur eine Hemmung von Thrombin, Faktor Xa, Plasmin und Trypsin beschrieben ist, finden sich bei Stürzebecher et al. (1997) auch Angaben zur Hemmung von uPA. Bei Nα-2-Naphthylsulfonyl-, Nα-2-(2,2,5,7,8-Pentamethylchroman-6-yl)sulfonyl-und Nα-2-Campher-10-yl-sulfonyl-substituierten 3-Amidinophenylalaninpiperaziden wird für uPA ein Kᵢ-Wert von 28 bis 140 µmol/l gefunden, der um drei Größenordnungen höher als die Inhibitionskonstante für Thrombin ist.

Es wurde auch gefunden, dass an Position 2 mit einem Phenylrest substituierte 3-Amidinophenylalaninderivate selektive und *in vivo* wirksame Hemmstoffe von uPA darstellen (WO 00/04954). Weiterhin wurde gefunden, dass diese Substanzen eine hohe Selektivität für Lymphgewebe aufweisen und sich daher als Mittel zum Targeting von Lymphzellen, beispielsweise zur Behandlung von malignen Erkrankungen des Lymphgewebes, wie etwa Lymphomen, eignen.

Es besteht aber nach wie vor ein Bedarf an der Entwicklung weiterer Hemmstoffe mit einer höheren Selektivität für uPA, um die Rolle, die uPA und uPAR bei verschiedenen Krankheiten, speziell bei der Tumorausbreitung und Metastasierung spielen, weiter aufzuklären.

Überraschenderweise konnte nun gefunden werden, das ein Austausch der Amidinofunktion durch die Guanidinofunktion bei den Phenylalaninderivaten die Hemmwirkung bezüglich uPA nicht nachteilig beeinflusst und derartige Inhibitoren darüber hinaus eine hohe Selektivität für uPA aufweisen.

Die vorliegende Erfindung betrifft von 3-Guanidinophenylalanin abgeleitete neue Urokinase-Inhibitoren der allgemeinen Formel I, 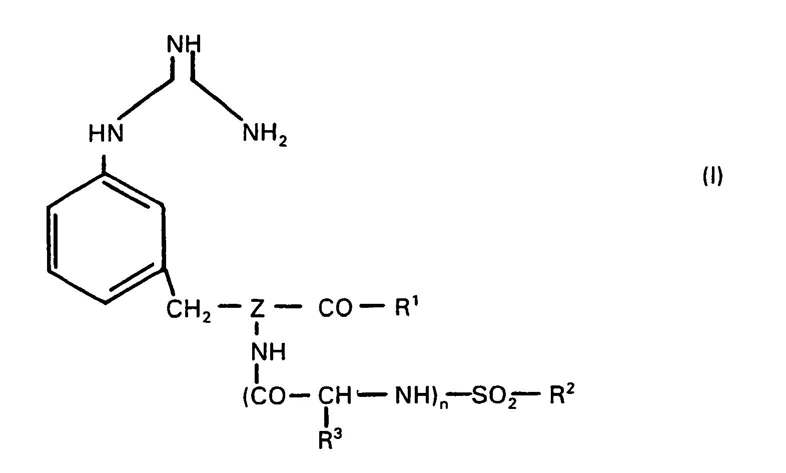 die als Racemate sowie als L- bzw. D-konfigurierte Verbindungen vorliegen und in denen
R1
   (a) OH oder OR⁴ ist, wobei R⁴ ein gegebenenfalls z.B. mit Hydroxyl, Carboxyl, Sulfonyl, Nitro, Cyano, Oxo oder/und Halogen substituiertes, verzweigtes oder unverzweigtes C₁-C₈-Alkyl, C₃-C₈-Cycloalkyl oder Aralkyl, z.B. Benzyl oder Phenylethyl ist,
   (b) eine Gruppe der Formel 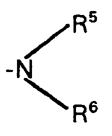 darstellt, in welcher R⁵ und
      R⁶ beliebige mit der Gesamtstruktur kompatible Reste sind, wobei insbesondere
         (i) R⁵ und R⁶ H sind,
         (ii) R⁵ H ist und R⁶ ein gegebenenfalls z.B. mit Hydroxyl, Carboxyl, Sulfonyl, Nitro, Cyano, Oxo oder/und Halogen substituiertes verzweigtes oder unverzweigtes C₁-C₈-Alkyl, Aralkyl, z.B. Benzyl oder Phenylethyl, oder C₅-C₈ Cycloalkyl ist,
         (iii) R⁵ und R⁶ jeweils unabhängig ein gegebenenfalls z.B. mit Hydroxyl oder/und Halogen substituiertes, unverzweigtes oder verzweigtes C₁-C₄ Alkyl sind oder
         (iv) R⁵ H ist und R⁶ -NH₂ oder eine insbesondere mit Aryl oder Heteroaryl substituierte Aminogruppe ist,
         (v) R⁵ H oder ein gegebenenfalls z.B. mit Hydroxyl oder/und Halogen substituiertes, unverzweigtes oder verzweigtes C₁-C₄ Alkyl ist und R⁶ der Rest einer Aminosäure, z.B. einer α-, ß- oder ω-Aminocarbon-oder Aminosulfonsäure, oder der Rest eines Peptids z.B. mit einer Länge bis zu 50 Aminosäuren oder eines Polypeptids z.B. mit einer Länge von mehr als 50 Aminosäuren bis 1.000 Aminosäuren ist,
   (c) eine Gruppe der Formel 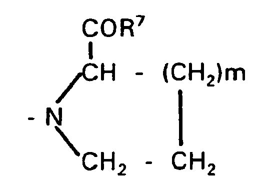 darstellt, in welcher m die Zahl 1 oder 2 bezeichnet, und in welcher eine oder mehrere der Methylengruppen gegebenenfalls z.B. mit einem Hydroxyl-, Carboxyl-, C₁-C₄-Alkyl- oder Aralkylrest, z.B. Benzyl oder Phenylethyl, substituiert sind, wobei die Gruppe (c) racemisch oder D-bzw. L-konfiguriert ist, und R⁷ die Bedeutung von R¹ in den Ziffern (a), (b) und (f) aufweist,
   (d) eine Gruppe der Formel 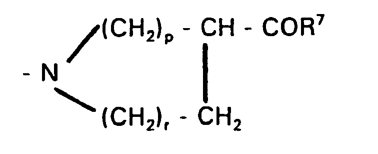 darstellt, in welcher p = r = 1, p = 1 und r = 2 oder p = 2 und r = 1 sind und in welcher eine oder mehrere der Methylengruppen gegebenenfalls z.B. mit einem Hydroxyl-, Carboxyl-, C₁-C₄-Alkyl- oder Aralkylrest, z.B. Benzyl oder Phenylethyl, substitutiert sind, und R⁷ die Bedeutung von R¹ in Ziffer (a), (b) und (f) aufweist,
   (e) eine Piperidylgruppe darstellt, die gegebenenfalls in einer der Stellungen 2, 3 und 4 z.B. mit einem C₁-C₄-Alkyl- , C₁-C₃ Alkoxy- oder Hydroxylrest substituiert ist, wobei gegebenenfalls an die heterocycloaliphatischen Ringe der Formeln (c), (d), (e) ein weiterer aromatischer oder cycloaliphatischer Ring, vorzugsweise Phenyl oder Cyclohexyl, in 2,3 oder 3,4 Stellung, bezogen auf das Heteroatom, ankondensiert ist,
   (f) eine Gruppe der Formel 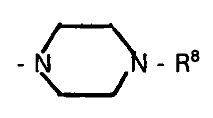 darstellt, in welcher R⁸
      (i) einen gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituierten C₁-C₆-Alkyl- oder Arylrest bedeutet, wie z.B. Phenyl, p-Halogenphenyl, Naphthyl,
      (ii) einen gesättigten oder ungesättigten, verzweigten oder unverzweigten C₁-C₆-Alkoxyrest bedeutet,
      (iii) einen Oxycarbonylrest -COOR' bedeutet, worin R' H oder eine Gruppe, wie etwa C₁-C₆-Alkyl, Aryl oder Aralkyl, gegebenenfalls substituiert mit C₁-C₃-Alkoxy, Hydroxyl, Carboxyl, Sulfonyl, Nitro, Cyano, Oxo oder/und Halogen, ist, z.B. einen Ethoxycarbonyl-, Phenoxycarbonyl- bzw. Benzyloxycarbonylrest, oder
      (iv) einen Aminocarbonylrest -CONR'R" bedeutet, worin R' und R" jeweils unabhängig H oder eine Gruppe, wie etwa C₁C₄-Alkyl, Aryl oder Aralkyl, gegebenenfalls substituiert mit C₁-C₃-Alkoxy, Hydroxyl, Carboxyl, Sulfonyl, Nitro, Cyano, Oxo oder/und Halogen, sind, z.B. Ethylaminocarbonyl,
   (g) einen Acylrest der Formel -COX darstellt, wobei X
      (i) H, einen gegebenenfalls z.B. mit Hydroxyl, Carboxyl, Sulfonyl, Nitro, Cyano, Oxo oder/und Halogen substituierten, unverzweigten oder verzweigten Alkylrest, vorzugsweise einen C₁-C₆-Alkylrest, insbesondere Methyl,
      (ii) einen gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituierten Aryl- oder Heteroarylrest, wie z.B. Phenyl, p-Halogenphenyl, Thienyl oder
      (iii) einen gegebenenfalls z.B. mit Hydroxyl, Carboxyl, Sulfonyl, Nitro, Cyano, Oxo oder/und Halogen substituierten Cycloalkylrest, vorzugsweise einen C₃-C₁₀-Cycloalkylrest bedeutet,
   (h) einen Aralkylrest, z.B. Benzyl oder Phenylethyl, darstellt, in dem der aromatische Rest gegebenenfalls z.B. mit einem Halogenatom, einer C₁-C₆-Alkyl-, C₁-C₃-Alkoxy-, Hydroxy-, Cyano-, Carboxyl-, Sulfonyl- oder Nitrogruppe substituiert ist,
   (i) einen Carbonsäureamidrest der Formel -CONR'R", einen Thiocarbonsäureamidrest -CSNR'R" oder einen Essigsäureamidrest -CH₂-CONR'R" darstellt, wobei
      (i) R' und R" H sind,
      (ii) R' und R" jeweils unabhängig C₁-C₄-Alkyl sind,
      (iii) R' H ist und R" C₁-C₄-Alkyl ist,
      (iv) R' H ist und R" Aryl, z.B. Phenyl, ist oder
      (v) R' und R" mit dem Stickstoffatom einen heterocycloaliphatischen Ring mit 5-7 Ringgliedern, der ein weiteres Heteroatom, z.B. N, O oder/und S tragen kann, bilden,
   (j) einen SO₂-Y-Rest darstellt, in dem Y
      (i) ein gegebenenfalls z.B. mit Hydroxyl, Carboxyl, Sulfonyl, Nitro, Cyano, Oxo oder/und Halogen substitutiertes C₁-C₈-Alkyl, vorzugsweise Methyl, Trifluormethyl, Trichlormethyl,
      (ii) ein gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituiertes Aryl oder Heteroaryl, wie z.B. Phenyl, 4-Methyl-phenyl, 2,4,6-Trimethyl-phenyl, 2,4,6-Triisopropyl-phenyl, 4-Methoxy-2,3,6-Trimethyl-phenyl, 2,2-Dimethyl-6-methoxy-chromanyl, 2,2,5,7,8-Pentamethyl-chromanyl, Anthrachinonyl, Naphthyl oder Chinolyl, bzw. O-Aryl, vorzugsweise O-Phenyl, oder O-Heteroaryl oder
      (iii) -NR'R" ist, wobei R' und R" jeweils unabhängig H oder C₁-C₃-Alkyl bedeuten,
   (k) einen cycloaliphatischen Ring mit 5 bis 8 C-Atomen darstellt, der gegebenenfalls z.B. mit einer C₁-C₆-Alkyl-, C₁-C₃-Alkoxy-, Halogen-, Hydroxyl- oder/und Oxogruppe substituiert ist,
   (l) einen gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituierten Heteroarylrest, wie z.B. Pyridyl oder Pyrimidyl, oder heterocycloaliphatischen Rest, beispielsweise N-Methylpiperidyl darstellt,
   (m) einen funktionalisierten Alkylrest der Formel -(CH₂)ₙ-X darstellt, wobei die Alkylkette unverzweigt oder verzweigt ist, n = 1 bis 8 bedeutet und der funktionelle Rest X
      (i) eine Hydroxylgruppe darstellt, deren H-Atom gegebenenfalls durch eine C₁-C₄-Alkyl-, Aralkyl-, z.B. Benzyl oder Phenylethyl, Aryl-, z.B. Phenyl, C₁-C₄ Hydroxyalkyl- oder Acylgruppe CO-Alkyl, (C₁-C₆), substituiert ist,
      (ii) ein Halogenatom bedeutet,
      (iii) eine tertiäre Aminogruppe der Formel -N(Alk)₂ darstellt, wobei die Alkylgruppen 1 bis 3 C-Atome sowie vorzugsweise die gleiche Bedeutung besitzen und das Stickstoffatom gegebenenfalls einem heterocycloaliphatischen Ring mit 5-7 Ringgliedern, der ein weiteres Heteroatom, z.B. N, O oder/und S tragen kann, angehört,
R² einen gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituierten Phenylrest, wie beispielsweise Phenyl, 4-Methyl-phenyl, 2,4,6-Trimethyl-phenyl, 2,4,6-Triisopropyl-phenyl, 4-Methoxy-2,3,6-trimethyl-phenyl darstellt,
R³ H oder verzweigtes bzw. unverzweigtes C₁-C₄-Alkyl ist und n 0 oder 1 bedeutet,
Z N oder CR⁹ bedeutet, wobei R⁹ H oder verzweigtes oder unverzweigtes C₁-C₄-Alkyl ist.

Die Verbindungen können auch als Salze, vorzugsweise als physiologisch verträgliche Säuresalze, z.B. als Salze von Mineralsäuren, besonders bevorzugt als Hydrochloride, oder als Salze von geeigneten organischen Säuren vorliegen.

Von den in den allgemeinen Ansprüchen definierten Verbindungen sind solche, bei denen R¹ einer Gruppe der Formeln (b), (d) und (f) entspricht, R² einen einfach, zweifach oder dreifach alkylsubstituierten Phenylrest, insbesondere einen 2,4,6-substituierten Phenylrest, z.B. einen 2,4,6-Triisopropylphenyl-Rest darstellt, und n = 0 ist, von besonderer Bedeutung. Weiterhin bevorzugt sind Verbindungen, bei denen Z CH oder N ist. R¹ ist besonders bevorzugt ein 4-Ethoxycarbonylpiperazid-Rest oder ein 4-Ethylaminocarbanylpiperazid-Rest.

Die vorliegende Erfindung betrifft die Verbindungen der allgemeinen Formel (I) als solche, als Wirkstoffe in pharmazeutischen Zusammensetzungen und zur Verwendung zur Prävention oder Behandlung von mit Urokinase oder/und Urokinase-Rezeptor, insbesondere mit deren Überexpression assoziierten Erkrankungen.

Die Verbindungen der allgemeinen Formel I können in prinzipiell bekannter Weise, wie z.B. in WO 92/08709 und WO 94/18185 beschrieben, hergestellt und auf ihre biologische *in vitro* Aktivität getestet werden.

(L)-, (D)- oder (D,L)-3-Nitrophenylalanin wird mit einem entsprechenden Sulfonylchlorid oder einer sulfonylierten Aminosäure bzw. deren Halogenid in Gegenwart einer Base zu einer Verbindung der allgemeinen Formel I mit Nitrofunktion, in der R¹ = OH ist und R² sowie R³ und n den in den allgemeinen Ansprüchen definierten Bedeutungen entspricht, umgesetzt. Die erhaltenen Verbindungen werden durch Veresterung mit einem entsprechenden Alkohol unter säurekatalytischen Bedingungen zu Verbindungen der allgemeinen Formel I überführt, wobei R¹ = (a) bedeutet. Nach einem in der Peptidchemie üblichen Verfahren, z.B. DCC-Verfahren in Gegenwart von HOBt, sind durch Umsetzung der Carbonsäuren der allgemeinen Formel I (R¹ = -OH) mit einem Nukleophil der Strukturen (b), (e) und (f) die Verbindungen mit entsprechendem R¹ der allgemeinen Formel I darstellbar. Für die Synthese von Verbindungen mit R¹ = (c) und (d) werden zunächst die Carbonsäuren der allgemeinen Formel I mit R¹ = OH mit cycloaliphatischen Aminosäureestern der Strukturen (c) und (d), wobei R⁷ vorzugsweise -OCH₃ bzw. -OC₂H₅ bedeutet, umgesetzt, die erhaltenen Carbonsäurester unter milden sauren oder alkalischen Bedingungen zu den entsprechenden Carbonsäuren hydrolysiert, die nachfolgend in bereits beschriebener Weise verestert oder mit Nukleophilen der Struktur (b), (e) und (f) umgesetzt werden können, wobei Verbindungen der allgemeinen Formel I mit R¹ = (c) sowie (d) und R⁷ = (a), (b), (e) und (f) erhalten werden.

Zielverbindungen der allgemeinen Formel I mit Guanidinstruktur sind aus den Nitroverbindungen in bekannter Weise erhältlich, wobei in der Regel durch Reduktion der Nitrogruppe zunächst die entsprechenden Amine erhalten werden, die durch Umsetzung mit einem geeigneten Guanidinylierungsreagenz, wie beispielsweise Guanylpyrazol, in die Guanidinoverbindungen überführt werden.

Die erfindungsgemäßen Urokinaseinhibitoren können gegebenenfalls zusammen mit mindestens einem geeigneten pharmazeutischen Hilfs- oder Trägerstoff zur Herstellung von Arzneimitteln, z.B. von oral, subkutan oder intravenös verabreichbaren Arzneimitteln zur Tumorbekämpfung oder in der Diagnostik verwendet werden. Ebenfalls möglich ist die Verabreichung in Kombination mit anderen Wirkstoffen, z.B. anderen Urokinaseinhibitoren, wie etwa Antikörpern oder/und Peptiden.

Die Arzneimittel zur Tumorbekämpfung bei Menschen und Tieren können topisch, oral, rektal oder parenteral, z.B. subkutan oder intravenös, in Form von Tabletten, Dragees, Kapseln, Pellets, Suppositorien, Lösungen oder transdermalen Systemen, wie Pflastern, verabreicht werden.

Besonders bevorzugt handelt es sich bei den Verbindungen der Formel (I) um Nα-(2,4,6-Triisopropyl-phenylsulfonyl)-3-guanidino-(D,L)-phenylalanin-4-ethoxycarbonyl-piperazid, Nα-(2,4,6-Triisopropyl-phenylsulfonyl)-3-guanidino-(D,L)-phenylalanin-4-ethylamino-piperazid bzw. um die L-Entantiomere davon oder um pharmazeutisch verträgliche Salze dieser Verbindungen, z.B. die Hydrochloride. Diese Substanzen haben ein gutes Löslichkeitsverhalten.

Die erfindungsgemäßen Verbindungen sind in der Lage, hocheffizient das Wachstum oder/und die Ausbreitung von malignen Tumoren zu hemmen, z.B. die Tumorausbreitung beim Pankreaskarzinom, das Tumorwachstum des Mammakarzinoms sowie die Metastasierung von Tumoren. Dabei können die uPA-Inhibitoren gegebenenfalls zusammen mit anderen Antitumormitteln oder mit anderen Behandlungsarten, z.B. Bestrahlung oder chirurgischen Eingriffen, eingesetzt werden. Weiterhin sind die erfindungsgemäßen Inhibitoren auch für andere uPA- oder/und uPARassoziierte Erkrankungen wirksam (z.B. bei der Verhinderung der Blasenbildung bei der Hauterkrankung Pemphigus vulgaris).

Erfindungsgemäße uPA Inhibitoren sind vorzugsweise dadurch gekennzeichnet, dass sie einen mindestens 2-fach, vorzugsweise mindestens 5-fach und besonders bevorzugt mindestens 10-fach geringeren Kᵢ-Wert für uPA gegenüber Plasmin, Thrombin oder/und tPA aufweisen. Daher ist bemerkenswert, dass die erfindungsgemäßen Verbindungen die Blutgerinnung nur geringfügig beeinflussen, und somit eine überraschende Selektivität aufweisen.

Die erfindungsgemäßen Substanzen der Formel I können in Form von Konjugaten mit physiologisch wirksamen Substanzen eingesetzt werden, z.B. mit Radiomarkierungen oder mit zytotoxischen Mitteln, z.B. Chemotherapeutika, wie cis-Platin, Carboplatin, 5-Fluor-Uracil, Doxorubicin, Epirubicin oder Taxol, oder Peptiden. Weiterhin können die Substanzen auch in die Membran von Trägervesikeln, z.B. Liposomen, eingebaut werden und somit ein Targeting von in den Trägervesikeln eingeschlossenen Wirksubstanzen, z.B. zytotoxischen Mitteln, wie etwa Doxorubicin, ermöglichen.

Die Erfindung soll an dem folgenden Beispiel und der Abbildung näher erläutert werden. Es zeigt:
- **Figur 1**: ein Syntheseschema der erfindungsgemäß bevorzugten Substanz Nα-(2,4,6-Triisopropyl-phenylsulfonyl)-3-guanidino- (L)-phenylalanin-4-ethoxycarbonyl-piperazin.

### Beispiel 1

### Synthese von Nα-2,4,6-Triisopropyl-phenylsulfonyl-3-guanidino-(L)-phenylalanin-4-ethoxycarbonyl-piperazin-hydrochlorid

### 1.1 N-Acetyl-(D/L)-13-nitro-phenylalanin)

Eine Lösung von 3-Nitrobenzylbromid (7,5 g; 35,7 mmol), Acetamidomalonsäurediethylester (7,54 g; 35,7 mmol) und Kaliumiodid (0,3 g; 1,79 mmol) in Dioxan abs. wird unter Argon bei 80 °C gerührt, während man eine Natriumethanolat-Lösung (25 ml, 37,5 mmol) über 1,5 h zutropft. Die Reaktionsmischung wird 2 h refluxiert und über Nacht bei RT gerührt. Nach Zugabe von 3M NaOH (24 ml) bei 80°C wird weitere 4 h bei 95 °C gerührt. Das Dioxan wird abdestilliert und die wäßrige Phase 3x mit Ethylacetat (30 ml) extrahiert. Anschließend wird die wässrige Phase mit 1 M HCl auf pH 1 angesäuert und 3x mit Ethylacetat (30 ml) extrahiert. Beim langsamen Eindampfen der 3 letzten vereinigten Ethylacetatphasen kristallisiert das Produkt aus. Es wird abfiltriert und im Vakuum getrocknet.

Ausbeute: 6 g (69 %), ESI-MS: m/z: 253,3 (M+H)⁺; berechnet für C₁₁H₁₂N₂O₅: 252,2

### 1.2 (L)-(3-Nitro-phenylalanin)

Eine Lösung von N-Acetyl-(D/L)-(3-nitro-phenylalanin) (1.1) (5,75 g; 22,8 mmol) in 350ml Wasser wird mit 1 M NaOH auf pH 7,5 eingestellt. Nach Zugabe von Amano Acylase (0,25 g) wird die Mischung bei 37-38 °C 6 Tage langsam gerührt. Anschließend wird mit 1 M HCl auf pH 3 angesäuert und 3x mit Ethylacetat (je 100 ml) extrahiert und die organische Phase verworfen. Der pH-Wert der wässrigen Lösung wird mit 1 M NaOH auf 6,8 eingestellt, das Wasser sukzessive abdestilliert, das ausgefallene Produkt abfiltriert und getrocknet.

Ausbeute: 1,7 g (35 %), ESI-MS: m/z: 211,3 (M+H)⁺; berechnet für C₉H₁₀N₂O₄: 210,2

### 1.3 N-Triisopropylphenylsulfonyl-(L)-(3-nitro-phenylalanin)

Zu einer Lösung von (L)-(3-nitro-phenylalanin)(1.2) (1,7 g; 8,09 mmol) in einer Mischung aus 1 M NaOH (8 ml) und Dioxan (15 ml) wird parallel 1 M NaOH (8 ml) und ein Lösung von Triisopropylphenylsulfonylchlorid (2,42 g; 7,99 mmol) in Dioxan (15 ml) während 1,5 h zugetropft. Anschließend wird die Reaktionsmischung über Nacht bei RT gerührt. Nach Abziehen des Lösungsmittels wird der Rückstand in Ethylacetat aufgenommen (50 ml) und 3x mit 5 % KHSO₄-Lösung (je 25 ml) und 3x mit Wasser (je 20 ml) gewaschen. Nach Abziehen des Lösungsmittels, wird das Produkt im Hochvakuum getrocknet.

Ausbeute: 3,52 g (92 %), HPLC-Reinheit ca. 80 %; ESI-MS: m/z: 477,7 (M+H)⁺; berechnet für C₂₄H₃₂N₂O₆S₁: 476,6

### 1.4 N-Triisopropylphenylsulfonyl-(L)-(3-nitro-phenylalanin)-4-ethyloxy-carbonyl-piperazid

Eine Lösung von N-Triisopropylphenylsulfonyl-(L)-(3-nitro-phenylalanin) (1.3) (3, 52 g; 7,39 mmol), 1-Ethyloxycarbonylpiperazin (1,19 g; 7,47 mmol) und HOBt (1, 53 g; 11,3 mmol) in absolutem DMF (15 ml) wird auf 0 °C gekühlt und eine Lösung von DCC (1,7 g; 3,91 mmol) in DMF (10 ml) zugetropft. Der Reaktionsansatz wird 18 h bei RT gerührt. Nach Einengen des Lösungsmittels im Vakuum auf ca. 7 ml wird mit Ethylacetat verdünnt (50 ml) und 3x mit 5 % KHSO₄-Lösung und 3x mit Wasser gewaschen. Nach Abziehen des Lösungsmittels wird das Rohprodukt durch Flash Chromatographie über eine Kieselgelsäule (Gradient: Petrolether:Methyl-tert-Butylether (MTBE) 1:1 bis MTBE:Ethylacetat 1:1) gereinigt.

Ausbeute: 1,45 g (32 %), ESI-MS: m/z: 617,9 (M+H)⁺; berechnet für C₃₁H₄₄N₄O₇S₁: 616, 8

### 1.5 N-Triisopropylphenylsulfonyl-(L)-(3-amino-phenylalanin)-4-ethyloxy-carbonylpiperazid

Eine Lösung von N-Triisopropylphenylsulfonyl-(L)-(3-nitro-phenylalanin)-4-ethyloxycarbonyl-piperazid (1.4) (1,4 g; 2,27 mmol) in Ethanol wird bei RT 8 h über 0,15 g 10 % Pd auf Aktivkohle hydriert. Nach Abfiltrieren des Katalysators wird das Lösungsmittel im Vakuum abgezogen und das Produkt im Vakuum getrocknet.

Ausbeute: 1,35 g (98 %), ESI-MS: m/z: 587,9 (M+H)⁺; berechnet für C₃₁H₄₆N₄O₅S₁: 586,8

### 1.6 N-Triisopropylphenylsulfonyl-(L)-(3-(N'N"bis-BOC-guanidino)-phenylalanin)-4-ethyloxy-carbonylpiperazid

Eine Lösung von N-Triisopropylphenylsulfonyl-(L)-(3-amino-phenylalanin)-4-ethyloxy-carbonylpiperazid (1.5) (0,5 g; 0,85 mmol) und N,N'-Di-BOC-guanyl-pyrazol (0,27 g; 0,85 mmol) in Methylenchlorid (10 ml) wird 6 d bei RT unter Argon gerührt. Nach Abziehen des Lösungsmittels wird der Rückstand in Ethylacetat aufgenommen und 3x mit 5 % KHSO₄-Lösung und 3x mit 5 % NaHCO₃-Lösung (je 15 ml) extrahiert. Nach Abziehen des Lösungsmittels wird das Rohprodukt chromatographisch über eine Kieselgelsäule gereinigt (Gradient Petrolether (PE)/Ethylacetat (EE) 4:1 bis PE/EE 7:3).

Ausbeute: 0,6 g (85 %), ESI-MS: m/z: 829,9 (M+H)⁺; berechnet für C₄₂H₆₄N₆O₉S₁: 829,1

### 1.7 N-Triisopropylphenylsulfonyl-(L)-(3-guanidino-phenylalanin)-4-ethyloxy-carbonylpiperazid

N-Triisopropylphenylsulfonyl-(L)-(3-(N'N"bis-BOC-guanidino)-phenylalanin)-4-ethyloxy-carbonylpiperazid (1.6) (0,5 g; 0,603 mmol) wird durch Rühren in 4M HCl in Dioxan (8 ml) entschützt. Das Lösungsmittel wird abgezogen, der Rückstand in wenig Methanol aufgenommen und in MTBE (35 ml) gegossen, wobei das Produkt ausfällt und anschließend abfiltriert und im Vakuum getrocknet wird. Ausbeute 0,18 g (49 %), ESI-MS: m/z: 629,5 (M+H)⁺; berechnet für C₃₂H₄₈N₆O₅S₁: 628,8; HPLC: t_{R} = 6,8 min

Die Charakterisierung der Verbindungen erfolgte massenspektrometrisch mittels eines Waters ZQ 2000 ESI-MS Massenspektrometer (Waters GmbH, Eschborn, Deutschland); eine Reinheitsprüfung erfolgte mittels HPLC mit einer X-Terra Säule C8, 150 x 2,1 mm Ø (Waters GmbH, Eschborn, Deutschland), Gradient: Wasser/Methanol 50:50 bis 5:95 in 15 min.

### Beispiel 2

### Synthese von Nα-2,4,6-Triisopropyl-phenylsulfonyl-3-guanidino-(L)-phenylalanin-4-ethylaminocarbonyl-piperazin-hydrochlorid

Die Synthese erfolgte analog wie in Beispiel 1 beschrieben, abgesehen davon, dass 1-Ethylaminocarbonylpiperazin anstelle von 1-Ethyloxycarbonylpiperazin verwendet wurde.

### Beispiel 3

### In vitro Hemmung von Urokinase durch ausgewählte Verbindungen der Formel I

| **Konfiguration** | **R¹** | **R²** | **n** | **Kᵢµmol/l** |
|---|---|---|---|---|
| L | 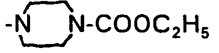 | TIPP | O | 0,47 |

Abkürzungen: TIPP - 2,4,6-Triisoprophylphenyl

### Bestimmung der Hemmwirkung

Zur Bestimmung der Inhibitoraktivität wurden 200 µl Tris-Puffer (0,05 mol/l, den Inhibitor enthaltend, 0,154 mol/l NaCl, 5% Ethanol pH 8,0), 25µl Substrat (Pefachrome UK oder Bz-βaAla-Gly-Arg-pNA in H₂O; Pentapharm Ltd., Basel, Schweiz) und 50 µl sc-Urokinase (Ribosepharm GmbH, Haan, Deutschland) bei 25°C inkubiert. Nach 3 min. wurde die Reaktion durch Zugabe von 25 µl Essigsäure (50%) unterbrochen und die Absorption bei 405 nm mittels Microplate Reader (MR 5000, Dynatech, Denkendorf, Deutschland) bestimmt. Die Kᵢ-Werte wurden nach Dixon durch lineare Regression mittels eines Computerprogramms ermittelt. Die Kᵢ-Werte sind das Mittel aus mindestens 3 Bestimmungen, die Standardabweichung lag unter 25%.

### Beispiel 4

### In vitro Hemmung verschiedener Serinproteasen vom Trypsin-Typ durch (Nα-(2,4,6-Triisopropyl-phenylsulfonyl)-(L)-3-guanidino-phenylalanin-4-ethoxycarbonyl-piperazid (uPA-Inhibitor)

| | Kᵢ[µmol/l] |
|---|---|
| **Enzym** | **uPA-Inh.** |
| Urokinase | 0,47 |
| Plasmin | 3,8 |
| Thrombin | ≥ 12 |

Die Bestimmung der Hemmwirkung für die verwendeten Enzyme erfolgte nach dem in Beispiel 3 beschriebenen Prinzip.

Aus den oben angegebenen Werten ist ersichtlich, dass der erfindungsgemäße uPA-Inhibitor überraschenderweise einen um mehr als den Faktor 5 kleineren Kᵢ-Wert als Plasmin und einen um mehr als den Faktor 10 kleineren Kᵢ-Wert für Urokinase als für Thrombin aufweist. Somit eigenen sich die erfindungsgemäßen Substanzen als selektive Urokinaseinhibitoren.

## Patentansprüche

1. Verwendung von Verbindungen der Formel (I) 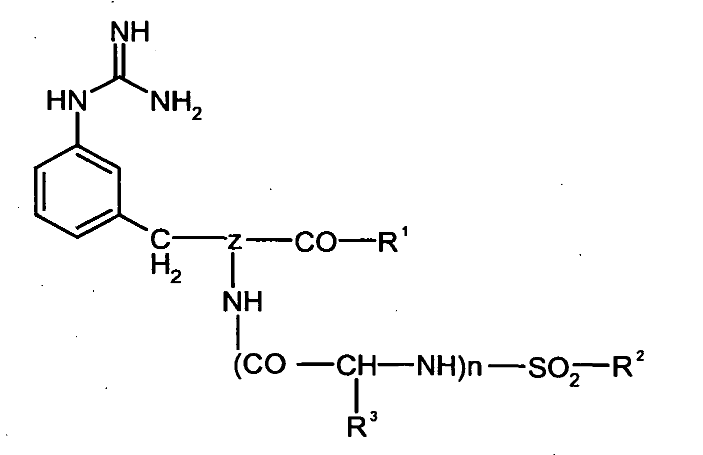 die als Racemate sowie als D- oder L-konfigurierte Verbindungen vorliegen können und in denen
R1
(a) OH oder OR⁴ ist, wobei R⁴ ein gegebenenfalls mit Hydroxyl, Carboxyl, Sulfonyl, Nitro, Cyano, Oxo oder/und Halogen substituiertes, verzweigtes oder unverzweigtes C₁-C₈-Alkyl, C₃-C₈-Cycloalkyl oder Benzyl oder Phenylethyl ist,
(b) eine Gruppe der Formel 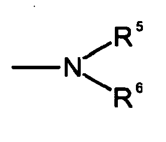 darstellt, wobei
(i) R⁵ und R⁶ H sind,
(ii) R⁵ H ist und R⁶ ein gegebenenfalls mit Hydroxyl, Carboxyl, Sulfonyl, Nitro, Cyano, Oxo oder/und Halogen substituiertes verzweigtes oder unverzweigtes C₁-C₈-Alkyl, Benzyl oder Phenylethyl oder C₅-C₈-Cycloalkyl ist,
(iii) R⁵ und R⁶ jeweils unabhängig ein gegebenenfalls mit Hydroxyl oder/und Halogen substituiertes, unverzweigtes oder verzweigte C₁-C₄-Alkyl sind, oder
(iv) R⁵ H ist und R⁶ -NH₂ oder eine mit Aryl oder Heteroaryl substituierte Aminogruppe ist,
(v) R⁵ H oder ein gegebenenfalls mit Hydroxyl oder/und Halogen substituiertes, unverzweigtes oder verzweigtes C₁-C₄-Alkyl ist und R⁶ der Rest einer α-, β- oder w-Aminocarbon- oder Aminosulfonsäure oder der Rest eines Peptids mit einer Länge bis zu 50 Aminosäuren oder eines Polypeptids mit einer Länge von mehr als 50 Aminosäuren bis 1000 Aminosäuren ist,
(c) eine Gruppe der Formel 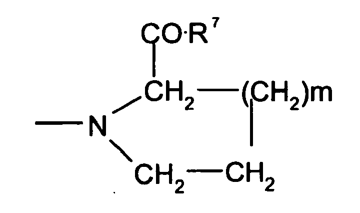 darstellt, in welcher m die Zahl 1 oder 2 bezeichnet, und in welcher eine oder mehrere Methylengruppen gegebenenfalls mit einem Hydroxyl-, Carboxyl-, C₁-C₄-Alkyl- oder Benzyl- oder Phenylethylrest substituiert sind, wobei die Gruppe (c) racemisch oder D- bzw. L-konfiguriert ist, und R⁷ die Bedeutung von R¹ in den Ziffern (a), (b) und (f) aufweist,
(d) eine Gruppe der Formel 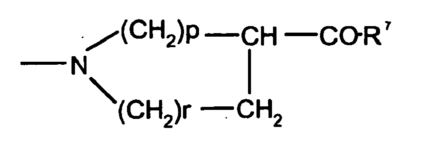 darstellt, in welcher p = r = 1, p = 1 und r = 2 oder p = 2 und r = 1 sind und in welcher eine oder mehrere der Methylengruppen gegebenenfalls mit einem Hydroxyl-, Carboxyl-, C₁-C₄-Alkyl- oder Benzyl- oder Phenyl-ethylrest substituiert sind, und R⁷ die Bedeutung von R¹ in Ziffer (a), (b) und (f) aufweist,
(e) eine Piperidylgruppe darstellt, die gegebenenfalls in einer der Stellungen 2, 3 und 4 mit einem C₁-C₄-Alkyl-, C₁-C₃-Alkoxy- oder Hydroxylrest substituiert ist, wobei gegebenenfalls an die heterocycloaliphatischen Ringe der Formeln (c), (d) (e) ein weiterer Phenyl-oder Cyclohexylring in 2,3 oder 3,4 Stellung bezogen auf das Heteroatom ankondensiert ist,
(f) eine Gruppe der Formel 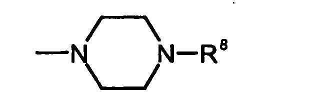 darstellt, in welcher R⁸
(i) einen gegebenenfalls mit C₁-C₆-Alkyl-, C₁-C₃-Alkoxy-, Hydroxyl, Carboxyl, Sulfonyl, Nitro, Cyano, Oxo oder/und Halogen substituierten C₁-C₆-Alkyl-, Phenyl-, p-Halogenphenyl-, Naphthylrest bedeutet,
(ii) einen gesättigten oder ungesättigten, verzweigten oder unverzweigten C₁-C₆-Alkoxyrest bedeutet,
(iii) einen Oxycarbonylrest -COOR' bedeutet, worin R' H oder eine C₁-C₆-Alkyl-, Aryl- oder Aralkylgruppe, gegebenenfalls substituiert mit C₁-C₃-Alkoxy, Hydroxyl, Carboxyl, Sulfonyl, Nitro, Cyano, Oxo oder/und Halogen ist, oder
(iv) einen Aminocarbonylrest -CONR'R" bedeutet, worin R' und R" jeweils unabhängig H oder eine C₁-C₄-Alkyl-, Aryl- oder Aralkylgruppe, gegebenenfalls substituiert mit C₁-C₃-Alkoxy, Hydroxyl, Carboxyl, Sulfonyl, Nitro, Cyano, Oxo oder/und Halogen, sind,
(g) einen Acylrest der Formel -COX darstellt, wobei X
(i) H, einen gegebenenfalls mit Hydroxyl, Carboxyl, Sulfonyl, Nitro, Cyano, Oxo oder/und Halogen substituierten, unverzweigten oder verzweigten C₁-C₆-Alkylrest,
(ii) einen gegebenenfalls mit C₁-C₆-Alkyl-, C₁-C₃-Alkoxy, Hydroxyl, Carboxyl, Sulfonyl, Nitro, Cyano, Oxo oder/und Halogen substituierten Phenyl-, p-Halogenphenyl-, Thienylrest oder
(iii) einen gegebenenfalls mit Hydroxyl, Carboxyl, Sulfonyl, Nitro, Cyano, Oxo oder/und Halogen substituierten C₃-C₁₀-Cycloalkylrest bedeutet,
(h) einen Benzyl- oder Phenylethylrest darstellt, in dem der aromatische Rest gegebenenfalls mit einem Halogenatom, einer C₁-C₆-Alkyl-, C₁-C₃-Alkoxy-, Hydroxy-, Cyano-, Carboxyl-, Sulfonyl- oder Nitrogruppe substituiert ist,
(i) einen Carbonsäureamidrest der Formel -CONR'R", einen Thiocarbonsäureamidrest -CSNR'R" oder einen Essigsäureamidrest -CH₂-CONR'R" darstellt, wobei
(i) R' und R" H sind,
(ii) R' und R" jeweils unabhängig C₁-C₄-Alkyl sind,
(iii) R' H ist und R" C₁-C₄-Alkyl ist,
(iv) R' H ist und R" Phenyl ist, oder
(v) R' und R" mit dem Stickstoffatom einen heterocycloaliphatischen Ring mit 5-7 Ringgliedern, der ein weiteres Heteroatom N, O oder/und S tragen kann, bilden,
(j) einen SO₂-Y-Rest darstellt, in dem Y
(i) ein gegebenenfalls mit Hydroxyl, Carboxyl, Sulfonyl, Nitro, Cyano, Oxo oder/und Halogen substituiertes C₁-C₈-Alkyl,
(ii) ein gegebenenfalls mit C₁-C₆-Alkyl, C₁-C₃-Alkoxy, Hydroxyl, Carboxyl, Sulfonyl, Nitro, Cyano, Oxo oder/und Halogen substituiertes Phenyl, 4-Methyl-phenyl, 2,4,6-Trimethylphenyl, 2,4,6-Triisopropylphenyl, 4-Methoxy-2,3,6-Trimethyl-phenyl, 2,2-Dimethyl-6-methoxy-chromanyl, 2,2,5,7,8-Pentamethyl-chromanyl, Anthrachinolyl, Naphthyl oder Chinolyl, O-Phenyl, oder Heteroaryl oder O-Heteroaryl, oder
(iii) -NR'R" ist, wobei R' und R" jeweils unabhängig H oder C₁-C₃-Alkyl bedeuten,
(k) einen cycloaliphatischen Ring mit 5 bis 8 C-Atomen darstellt, der gegebenenfalls mit einer C₁-C₆-Alkyl-, C₁-C₃-Alkoxy-, Halogen-, Hydroxyl- oder/und Oxogruppe substituiert ist,
(l) einen gegebenenfalls mit C₁-C₆-Alkyl, C₁-C₃-Alkoxy, Hydroxyl, Carboxyl, Sulfonyl, Nitro, Cyano, Oxo oder/und Halogen substituierten Pyridyl-, Pyrimidyl- oder N-Methylpiperidylrest darstellt,
(m) einen funktionalisierten Alkylrest der Formel -(CH₂)ₙ-X darstellt, wobei die Alkylkette unverzweigt oder verzweigt ist, n = 1 bis 8 bedeutet und der funktionelle Rest X
(i) eine Hydroxylgruppe darstellt, deren H-Atom gegebenenfalls durch eine C₁-C₄-Alkyl-, Benzyl- oder Phenylethyl-, Phenyl-, C₁-C₄-Hydroxyalkyl- oder Acylgruppe CO-Alkyl (C₁-C₆), substituiert ist,
(ii) ein Halogenatom bedeutet,
(iii) eine tertiäre Aminogruppe der Formel -N(Alk)₂ darstellt, wobei die Alkylgruppen 1 bis 3 C-Atome besitzen und das Stickstoffatom gegebenenfalls einem heterocycloaliphatischen Ring mit 5-7 Ringgliedern, der ein weiteres Heteroatom N, O oder/und S tragen kann, angehört,
R² einen gegebenenfalls mit C₁-C₆-Alkyl, C₁-C₃-Alkoxy, Hydroxyl, Carboxyl, Sulfonyl, Nitro, Cyano, Oxo oder/und Halogen substituierten Phenylrest oder 4-Methylphenyl, 2,4,6-Trimethylphenyl, 2,4,6-Triisopropylphenyl, 4-Methoxy-2,3,6-trimethyl-phenyl darstellt,
R³ H oder verzweigtes bzw. unverzweigtes C₁-C₄-Alkyl ist und n 0 oder 1 bedeutet,
Z N oder CR⁹ bedeutet, wobei R⁹ H oder verzweigtes bzw. unverzweigtes C₁-C₄-Alkyl ist, oder von Salzen der Verbindungen zur Herstellung eines Mittels für die Diagnostik, Therapie oder Prävention von Urokinase- oder Urokinaserezeptor-assoziierten Krankheiten.

2. Verwendung nach Anspruch 1,
**dadurch gekennzeichnet,**
**dass** R¹ eine Gruppe der Formeln (b), (d) und (f) ist, R² einen 2,4,6-Triisopropyl-phenyl-Rest darstellt und n = 0 ist.

3. Verwendung nach Anspruch 1 oder 2,
**dadurch gekennzeichnet,**
**dass** die Verbindung der Formel I Nα-(2,4,6-Triisopropylphenylsulfonyl)-3-guanidino-(D,L)-phenylalanin-4-ethoxycarbonyl-piperazid, Nα-(2,4,6-Triiso-propylphenylsulfonyl)-3-guanidino-(D,L)-phenylalanin-4-ethylaminocarbonyl-piperazid, das L-Enantiomer oder ein pharmazeutisch verträgliches Salz einer der Verbindungen ist.

4. Verwendung nach einem der Ansprüche 1 bis 3,
**dadurch gekennzeichnet,**
**dass** die Verbindungen in Form von physiologisch verträglichen Säuresalzen, insbesondere als Hydrochloride vorliegen.

5. Verwendung nach einem der Ansprüche 1 bis 4 zur Tumorbekämpfung.

6. Verwendung nach Anspruch 5 zur Bekämpfung von Mammakarzinom, Pankreaskarzinom und der Metastasenbildung.

7. Verwendung nach einem der Ansprüche 1 bis 4 zur Bekämpfung von Pemphigus vulgaris.

8. Verwendung nach einem der Ansprüche 1 bis 7,
**dadurch gekennzeichnet,**
**dass** die Verbindungen der Formel I als Konjugate mit weiteren pharmakologisch wirksamen Substanzen eingesetzt werden.

9. Verwendung nach einem der Ansprüche 1 bis 8,
**dadurch gekennzeichnet,**
**dass** die Verbindungen der Formel I in Kombination mit weiteren pharmakologisch wirksamen Substanzen eingesetzt werden.

10. Verwendung nach einem der Ansprüche 8 oder 9,
**dadurch gekennzeichnet,**
**dass** die Verbindungen als Konjugate mit Radiomarkierungen oder/und in Kombination mit zytotoxischen Substanzen eingesetzt werden.

11. Verwendung nach einem der Ansprüche 1 bis 10 zur Herstellung von oral, topisch, rektal oder parenteral verabreichbaren Arzneimitteln.

12. Verwendung nach einem der Ansprüche 1 bis 11 in Form von Tabletten, Dragees, Kapseln, Pellets, Suppositorien, Lösungen oder transdermalen Systemen, wie Pflastern.

13. Verwendung nach einem der Ansprüche 1-4, worin die Verbindung der Formel (I) ein Urokinase-Inhibitor ist.

14. Verbindungen der Formel (I) 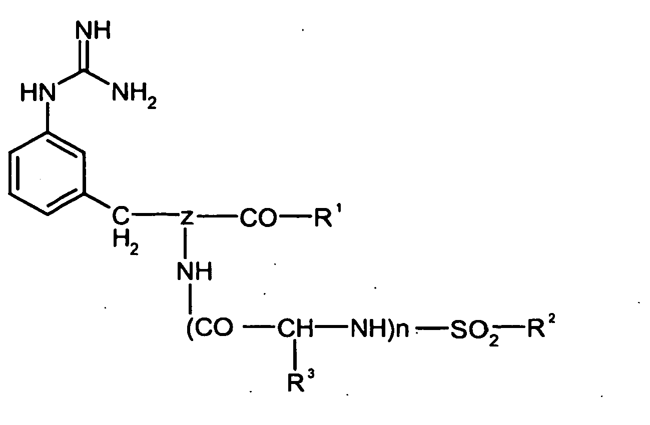 in denen R¹ und n wie in Anspruch 2 definiert sind, R³ und Z wie in Anspruch 1 definiert sind und R² ein 2,4,6-Triisopropylphenylrest ist.

15. Nα-(2,4,6-Triisopropylphenylsulfonyl)-3-guanidino-(D,L)-phenylalanin-4-ethoxycarbonyl-piperazid, Nα-(2,4,6-Triisopropylphenylsulfonyl)-3-guanidino-(D,L)-phenylalanin-4-ethylaminocarbonyl-piperazid, das L-Enantiomer davon oder ein pharmazeutisch verträgliches Salz einer der Verbindungen.

16. Pharmazeutische Zusammensetzung
**dadurch gekennzeichnet,**
**dass** sie als Wirkstoff eine oder mehrere Verbindungen nach Anspruch 14 oder 15, gegebenenfalls zusammen mit pharmazeutisch üblichen trägern oder/und Verdünnungsmitteln enthält.

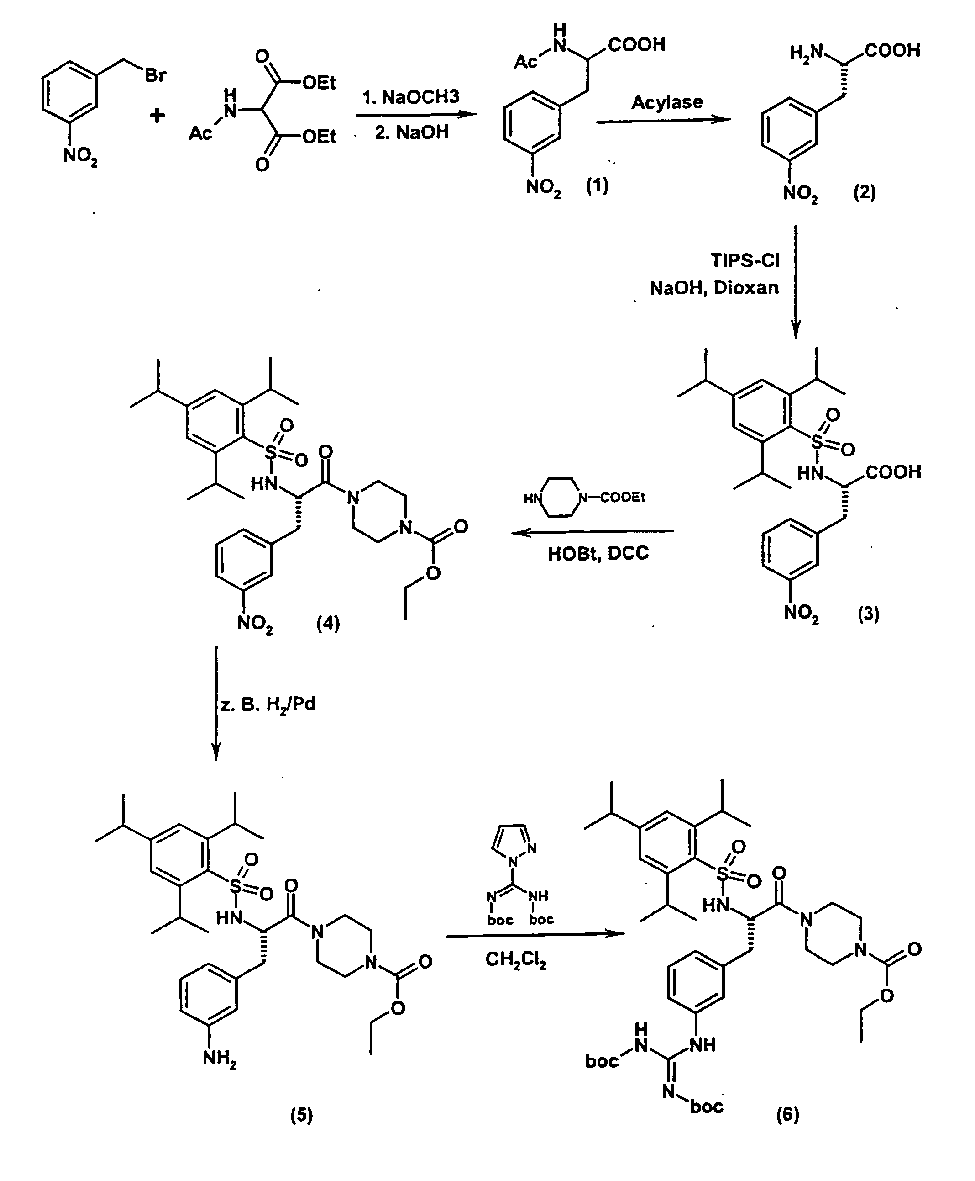

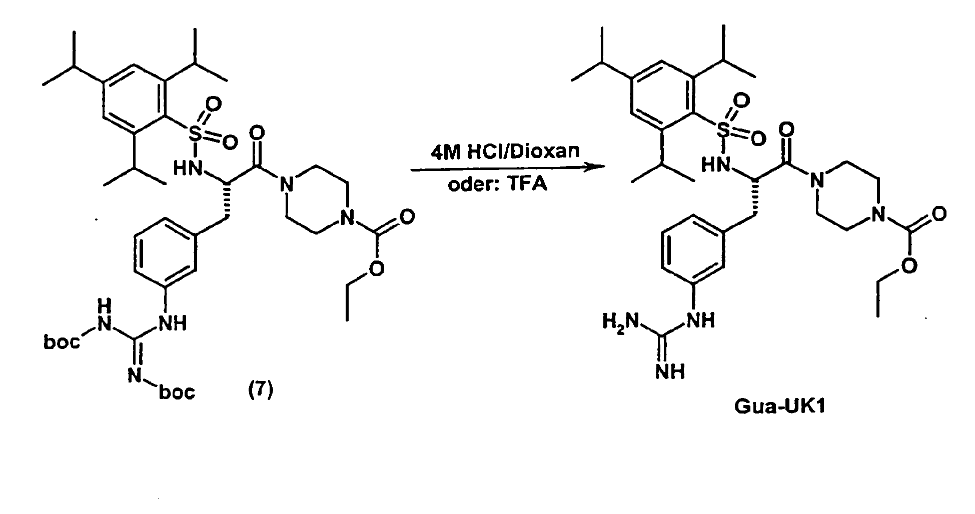

## Claims

1. Use of compounds of the formula (I) 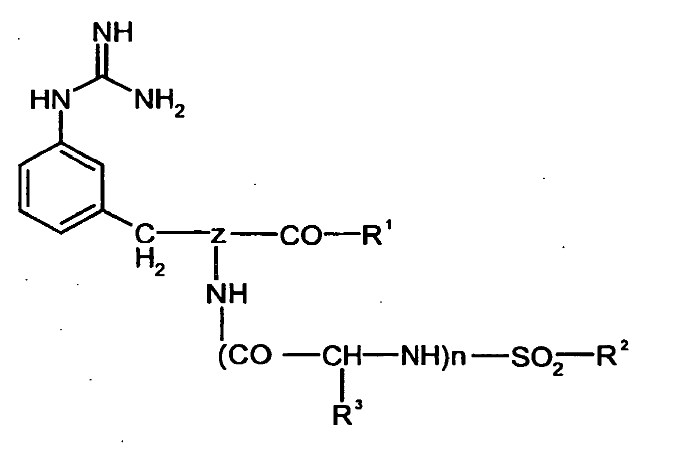 which may assume the form of racemates and of D or L configured compounds and in which
R¹
(a) is OH or OR⁴, wherein R⁴ is a branched or unbranched C₁-C₈ alkyl, C₃-C₈ cycloalkyl or benzyl or phenylethyl, optionally substituted with hydroxyl, carboxyl, sulfonyl, nitro, cyano, oxo and/or halogen,
(b) represents a group of the formula 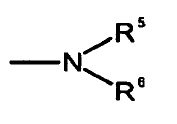 wherein
(i) R⁵ and R⁶ are H,
(ii) R⁵ is H and R⁶ is a branched or unbranched C₁-C₈ alkyl, benzyl or phenylethyl, or C₅-C₈ cycloalkyl, optionally substituted with hydroxyl, carboxyl, sulfonyl, nitro, cyano, oxo and/or halogen,
(iii) R⁵ and R⁶ are in each case independently an unbranched or branched C₁-C₄alkyl optionally substituted with hydroxyl and/or halogen or
(iv) R⁵ is H and R⁶ is -NH₂ or an amino group substituted with aryl or heteroaryl,
(v) R⁵ is H or an unbranched or branched C₁-C₄ alkyl optionally substituted with hydroxyl and/or halogen, and R⁶ is the residue of an α-, β- or ω-aminocarboxylic or aminosulfonic acid, or the residue of a peptide with a length of up to 50 amino acids or of a polypeptide with a length of more than 50 amino acids to 1000 amino acids,
(c) represents a group of the formula 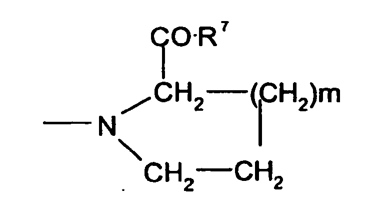 in which m denotes the number 1 or 2, and in which one or more of the methylene groups are optionally substituted with a hydroxyl, carboxyl, C₁-C₄ alkyl or benzyl or phenylethyl residue, wherein the group (c) is racemic or D or L configured, and R⁷ has the meaning of R¹ in indents (a) , (b) and (f),
(d) represents a group of the formula 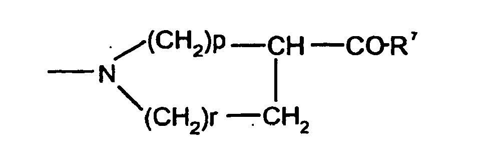 in which p = r = 1, p = 1 and r = 2 or p = 2 and r = 1 and in which one or more of the methylene groups are optionally substituted with a hydroxyl, carboxyl, C₁-C₄ alkyl or benzyl or phenylethyl residue, and R⁷ has the meaning of R¹ in indents (a) , (b) and (f),
(e) represents a piperidyl group, which is optionally substituted in one of positions 2, 3 and 4 with a C₁-C₄ alkyl, C₁-C₃ alkoxy or hydroxyl residue, wherein a further phenyl or cyclohexyl ring is optionally fused in 2,3 or 3,4 position, relative to the heteroatom, onto the heterocycloaliphatic rings of the formulae (c), (d), (e),
(f) represents a group of the formula 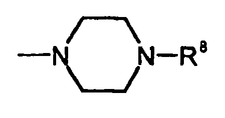 in which R⁸
(i) means a C₁-C₆ alkyl, phenyl, p-halophenyl, naphthyl residue optionally substituted with C₁-C₆ alkyl, C₁-C₃ alkoxy, hydroxyl, carboxyl, sulfonyl, nitro, cyano, oxo and/or halogen,
(ii) means a saturated or unsaturated, branched or unbranched C₁-C₆ alkoxy residue,
(iii) means an oxycarbonyl residue -COOR', in which R' is H or a C₁-C₆ alkyl, aryl or aralkyl group, optionally substituted with C₁-C₃ alkoxy, hydroxyl, carboxyl, sulfonyl, nitro, cyano, oxo and/or halogen, or
(iv) means an aminocarbonyl residue -CONR'R", in which R' and R" are in each case independently H or a C₁-C₄ alkyl, aryl or aralkyl group, optionally substituted with C₁-C₃ alkoxy, hydroxyl, carboxyl, sulfonyl, nitro, cyano, oxo and/or halogen,
(g) represents an acyl residue of the formula -COX, wherein X
(i) means H, an unbranched or branched C₁-C₆ alkyl residue optionally substituted with hydroxyl, carboxyl, sulfonyl, nitro, cyano, oxo and/or halogen,
(ii) means a phenyl, p-halophenyl, thienyl residue optionally substituted with C₁-C₆ alkyl, C₁-C₃ alkoxy, hydroxyl, carboxyl, sulfonyl, nitro, cyano, oxo and/or halogen or
(iii) means a C₃-C₁₀ cycloalkyl residue optionally substituted with hydroxyl, carboxyl, sulfonyl, nitro, cyano, oxo and/or halogen,
(h) represents a benzyl or phenylethyl residue, in which the aromatic residue is optionally substituted with a halogen atom, a C₁-C₆ alkyl, C₁-C₃ alkoxy, hydroxy, cyano, carboxyl, sulfonyl or nitro group,
(i) represents a carboxamide residue of the formula -CONR'R", a thiocarboxamide residue -CSNR'R" or an acetamide residue -CH₂-CONR'R", wherein
(i) R' and R" are H,
(ii) R' and R" are in each case independently C₁-C₄ alkyl,
(iii) R' is H and R" is C₁-C₄ alkyl,
(iv) R' is H and R" is phenyl, or
(v) R' and R" form, with the nitrogen atom, a heterocycloaliphatic ring with 5-7 ring members, which may bear a further heteroatom N, O and/or S,
(j) represents an SO₂-Y residue, in which Y
(i) means a C₁-C₈ alkyl optionally substituted with hydroxyl, carboxyl, sulfonyl, nitro, cyano, oxo and/or halogen,
(ii) means a phenyl optionally substituted with C₁-C₆ alkyl, C₁-C₃ alkoxy, hydroxyl, carboxyl, sulfonyl, nitro, cyano, oxo and/or halogen, 4-methylphenyl, 2,4,6-trimethylphenyl, 2,4,6-triisopropylphenyl, 4-methoxy-2,3,6-trimethylphenyl, 2,2-dimethyl-6-methoxychromanyl, 2,2,5,7,8-pentamethylchromanyl, anthraquinolyl, naphthyl or quinolyl, O-phenyl, or heteroaryl or 0-heteroaryl or
(iii) is -NRR", wherein R' and R" in each case independently mean H or C₁-C₃ alkyl,
(k) represents a cycloaliphatic ring with 5 to 8 C atoms which is optionally substituted with a C₁-C₆ alkyl, C₁-C₃ alkoxy, halogen, hydroxyl and/or oxo group,
(l) represents a pyridyl, pyrimidyl or N-methylpiperidyl residue optionally substituted with C₁-C₆ alkyl, C₁-C₃ alkoxy, hydroxyl, carboxyl, sulfonyl, nitro, cyano, oxo and/or halogen,
(m) represents a functionalised alkyl residue of the formula - (CH₂)ₙ-X, wherein the alkyl chain is unbranched or branched, n means 1 to 8 and the functional residue X
(i) represents a hydroxyl group, the H atom of which is optionally substituted by a C₁-C₄ alkyl, benzyl or phenylethyl, phenyl, C₁-C₄ hydroxyalkyl or acyl group CO-alkyl (C₁-C₆),
(ii) means a halogen atom,
(iii) represents a tertiary amino group of the formula -N(alk)₂, wherein the alkyl groups have 1 to 3 C atoms and the nitrogen atom optionally belongs to a heterocycloaliphatic ring with 5-7 ring members, which may bear a further heteroatom N, O and/or S,
R² represents a phenyl residue optionally substituted with C₁-C₆ alkyl, C₁-C₃ alkoxy, hydroxyl, carboxyl, sulfonyl, nitro, cyano, oxo and/or halogen, or 4-methylphenyl, 2,4,6-trimethylphenyl, 2,4,6-triisopropylphenyl, 4-methoxy-2,3,6-trimethylphenyl,
R³ is H or branched or unbranched C₁-C₄ alkyl and n means 0 or 1,
Z means N or CR⁹, wherein R⁹ is H or branched or unbranched C₁-C₄ alkyl,
or of salts of the compounds for producing an agent for the diagnosis, treatment or prevention of urokinase- or urokinase receptor-associated diseases.

2. Use according to claim 1, **characterised in that** R¹ is a group of the formulae (b), (d) and (f), R² represents a 2,4,6-triisopropylphenyl residue and n = 0.

3. Use according to claim 1 or claim 2, **characterised in that** the compound of the formula I is Nα-(2,4,6-triisopropylphenylsulfonyl)-3-guanidino-(D,L)-phenylalanine-4-ethoxycarbonylpiperazide, Nα-(2,4,6-triisopropylphenylsulfonyl)-3-guanidino-(D,L)-phenylalanine-4-ethylaminocarbonylpiperazide, the L enantiomer or a pharmaceutically acceptable salt of one of the compounds.

4. Use according to any one of claims 1 to 3, **characterised in that** the compounds assume the form of physiologically acceptable acid salts, in particular hydrochlorides.

5. Use according to any one of claims 1 to 4 for combating tumours.

6. Use according to claim 5 for combating mammary carcinoma, pancreatic carcinoma and metastasisation.

7. Use according to any one of claims 1 to 4 for combating pemphigus vulgaris.

8. Use according to any one of claims 1 to 7, **characterised in that** the compounds of the formula I are used as conjugates with further pharmacologically active substances.

9. Use according to any one of claims 1 to 8, **characterised in that** the compounds of the formula I are used in combination with further pharmacologically active substances.

10. Use according to either of claim 8 or claim 9, **characterised in that** the compounds are used as conjugates with radiolabelled substances and/or in combination with cytotoxic substances.

11. Use according to any one of claims 1 to 10 for producing orally, topically, rectally or parenterally administrable medicaments.

12. Use according to any one of claims 1 to 11 in the form of tablets, sugar-coated tablets, capsules, pellets, suppositories, solutions or transdermal systems, such as patches.

13. Use according to any one of claims 1-4, in which the compound of the formula (I) is a urokinase inhibitor.

14. Compounds of the formula (I) 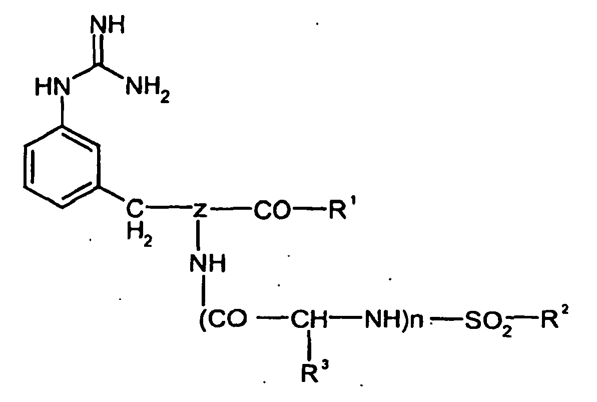 in which R¹ and n are as defined in claim 2, R³ and Z are defined as in claim 1 and R² is a 2,4,6-triisopropylphenyl residue.

15. Nα-(2,4,6-Triisopropylphenylsulfonyl)-3-guanidino-(D,L)-phenylalanine-4-ethoxycarbonylpiperazide, Nα-(2,4,6-triisopropylphenylsulfonyl)-3-guanidino-(D,L)-phenylalanine-4-ethylaminocarbonylpiperazide, the L enantiomer thereof or a pharmaceutically acceptable salt of one of the compounds.

16. A pharmaceutical composition **characterised in that** it contains as active ingredient one or more compounds according to claim 14 or claim 15, optionally together with pharmaceutically conventional excipients and/or diluents.

## Revendications

1. Utilisation de composés de formule (I) 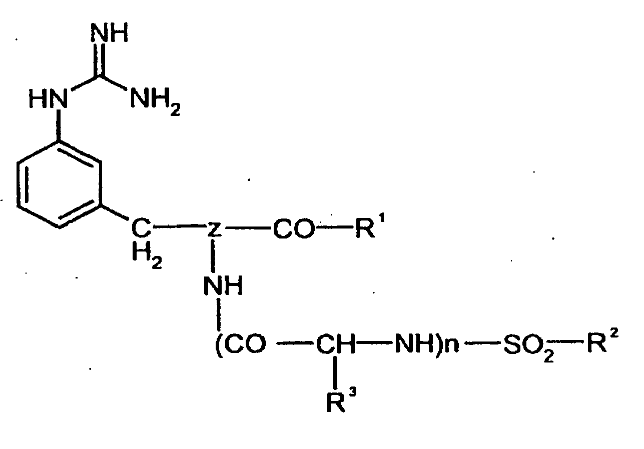 qui peuvent être sous forme de racémates ainsi que de composés de configuration D ou L et dans lesquels
R¹
(a) est OH ou OR⁴, où R⁴ est un C₁-C₈-alkyle ramifié ou non ramifié, C₃-C₈-cycloalkyle, benzyle ou phényléthyle éventuellement substitué avec hydroxyle, carboxyle, sulfonyle, nitro, cyano, oxo et/ou halogène,
(b) représente un groupe de formule 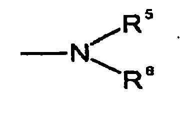 où
(i) R⁵ et R⁶ sont H,
(ii) R⁵ est H et R⁶ est un C₁-C₈-alkyle ramifié ou non ramifié, C₃-C₈-cycloalkyle, benzyle ou phényléthyle éventuellement substitué avec hydroxyle, carboxyle, sulfonyle, nitro, cyano, oxo et/ou halogène,
(iii) R⁵ et R⁶ sont chacun indépendamment un C₁-C₄-alkyle non ramifié ou ramifié, éventuellement substitué avec hydroxyle et/ou halogène, ou
(iv) R⁵ est H et R⁶ est -NH₂ ou un groupe amino substitué avec aryle ou hétéroaryle,
(v) R⁵ est H ou un C₁-C₄-alkyle non ramifié ou ramifié, éventuellement substitué avec hydroxyle et/ou halogène, et R⁶ est le résidu d'un acide α-, β- ou ω-aminocarboxylique ou aminosulfonique ou le résidu d'un peptide d'une longueur de jusqu'à 50 aminoacides ou d'un polypeptide d'une longueur de plus de 50 aminoacides à 1000 aminoacides,
(c) représente un groupe de formule 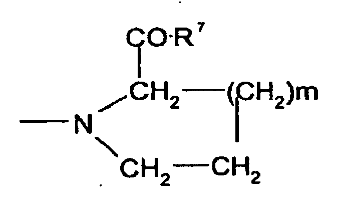 où m représente le nombre 1 ou 2, et où un ou plusieurs groupes méthylène sont éventuellement substitués avec un groupement hydroxyle, carboxyle, C₁-C₄-alkyle ou benzyle ou phényléthyle, où le groupe (c) est racémique ou de configuration D ou L, et R⁷ présente la signification de R¹ dans les numéros (a), (b) et (f),
(d) représente un groupe de formule 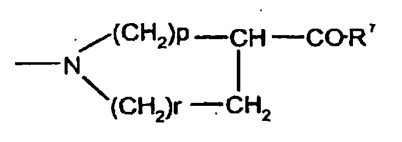 où p = r = 1, p = 1 et r = 2 ou p = 2 et r = 1 et où un ou plusieurs des groupes méthylène sont éventuellement substitués avec un groupe hydroxyle, carboxyle, C₁-C₄-alkyle ou benzyle ou phényléthyle, et R⁷ présente la signification de R¹ dans les numéros (a), (b) et (f),
(e) représente un groupe pipéridyle qui est éventuellement substitué dans l'une des positions 2, 3 et 4 avec un groupement C₁-C₄-alkyle, C₁-C₃-alcoxy ou hydroxyle, où éventuellement un autre cycle phényle ou cyclohexyle est condensé en position 2,3 ou 3,4 par rapport à l'hétéroatome sur les cycles hétérocycloaliphatiques des formules (c), (d), (e),
(f) représente un groupe de formule 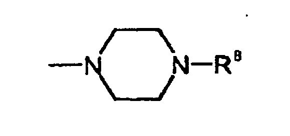 où R⁸
(i) représente un groupement C₁-C₆-alkyle, phényle, p-halogénophényle, naphtyle éventuellement substitué avec C₁-C₆-alkyle, C₁-C₃-alcoxy, hydroxyle, carboxyle, sulfonyle, nitro, cyano, oxo et/ou halogène,
(ii) un groupement C₁-C₆-alcoxy ramifié ou non ramifié, saturé ou insaturé,
(iii) un groupement oxycarbonyle -COOR' où R' est H ou un groupe C₁-C₆-alkyle, aryle ou aralkyle, éventuellement substitué avec C₁-C₃-alcoxy, hydroxyle, carboxyle, sulfonyle, nitro, cyano, oxo et/ou halogène, ou
(iv) un groupement aminocarbonyle -CONR'R" où R' et R" sont chacun indépendamment H ou un groupe C₁-C₄-alkyle, aryle ou aralkyle, éventuellement substitué avec C₁-C₃-alcoxy, hydroxyle, carboxyle, sulfonyle, nitro, cyano, oxo et/ou halogène,
(g) représente un groupement acyle de formule -COX où X
(i) représente H, un groupement C₁-C₆-alkyle non ramifié ou ramifié, éventuellement substitué avec hydroxyle, carboxyle, sulfonyle, nitro, cyano, oxo et/ou halogène,
(ii) un groupement phényle, p-halogénophényle, thiényle éventuellement substitué avec C₁-C₆-alkyle, C₁-C₃-alcoxy, hydroxyle, carboxyle, sulfonyle, nitro, cyano, oxo et/ou halogène, ou
(iii) un groupement C₃-C₁₀-cycloalkyle éventuellement substitué avec hydroxyle, carboxyle, sulfonyle, nitro, cyano, oxo et/ou halogène,
(h) représente un groupement benzyle ou phényléthyle dans lequel le groupement aromatique est éventuellement substitué avec un atome d'halogène, un groupe C₁-C₆-alkyle, C₁-C₃-alcoxy, hydroxyle, cyano, carboxyle, sulfonyle ou nitro,
(i) un groupement amide d'acide carboxylique de formule -CONR'R", un groupement amide d'acide thiocarboxylique -CSNR'R" ou un groupement amide d'acide acétique -CH₂-CONR'R", où
(i) R' et R" sont H,
(ii) R' et R" sont chacun indépendamment C₁-C₄-alkyle,
(iii) R' est H et R" est C₁-C₄-alkyle,
(iv) R' est H et R" est phényle, ou
(v) R' et R" forment avec l'atome d'azote un cycle hétérocycloaliphatique à 5-7 chaînons du cycle qui peut porter un autre hétéroatome N, O et/ou S,
(j) représente un groupement SO₂-Y où Y
(i) représente un C₁-C₈-alkyle éventuellement substitué avec hydroxyle, carboxyle, sulfonyle, nitro, cyano, oxo et/ou halogène,
(ii) un phényle, 4-méthyl-phényle, 2,4,6-triméthylphényle, 2,4,6-triisopropylphényle, 4-méthoxy-2,3,6-triméthyl-phényle, 2,2-diméthyl-6-méthoxy-chromanyle, 2,2,5,7,8-pentaméthyl-chromanyle, anthraquinolyle, naphtyle ou quinolyle, O-phényle ou hétéroaryle ou O-hétéroaryle éventuellement substitué avec C₁-C₆-alkyle, C₁-C₃-alcoxy, hydroxyle, carboxyle, sulfonyle, nitro, cyano, oxo et/ou halogène, ou
(iii) est -NR'R" où R' et R" représentent chacun indépendamment H ou C₁-C₃-alkyle,
(k) représente un cycle cycloaliphatique à 5 à 8 atomes C qui est éventuellement substitué avec un groupe C₁-C₆-alkyle, C₁-C₃-alcoxy, halogène, hydroxyle et/ou oxo,
(l) représente un groupement pyridyle, pyrimidyle ou N-méthylpipéridyle éventuellement substitué avec C₁-C₆-alkyle, C₁-C₃-alcoxy, hydroxyle, carboxyle, sulfonyle, nitro, cyano, oxo et/ou halogène,
(m) représente un groupement alkyle fonctionnalisé de formule -(CH₂)ₙ-X où la chaîne alkyle est non ramifiée ou ramifiée, n = 1 à 8 et le groupement fonctionnel X
(i) représente un groupe hydroxyle dont l'atome H est éventuellement substitué par un groupe C₁-C₆-alkyle, benzyle ou phényléthyle, phényle, C₁-C₄-hydroxyalkyle ou acyle CO-alkyle (C₁-C₆),
(ii) représente un atome d'halogène,
(iii) représente un groupe amino tertiaire de formule -N(Alk)₂ où les groupes alkyle possèdent 1 à 3 atomes C et l'atome d'azote appartient éventuellement à un cycle hétérocycloaliphatique à 5 à 7 membres du cycle, qui peut porter un autre hétéroatome N, O et/ou S,
R² représente un groupement phényle ou 4-méthylphényle, 2,4,6-triméthylphényle, 2,4,6-triisopropylphényle, 4-méthoxy-2,3,6-triméthyl-phényle éventuellement substitué avec C₁-C₆-alkyle, C₁-C₃-alcoxy, hydroxyle, carboxyle, sulfonyle, nitro, cyano, oxo et/ou halogène,
R³ est H ou C₁-C₄-alkyle ramifié ou non ramifié et n représente 0 ou 1,
Z représente N ou CR⁹ où R⁹ est H ou C₁-C₄-alkyle ramifié ou non ramifié,
ou de sels des composés pour produire un agent pour le diagnostic, la thérapie ou la prévention des maladies associées avec l'urokinase ou un récepteur d'urokinase.

2. Utilisation selon la revendication 1 **caractérisée en ce que** R¹ est un groupe des formules (b), (d) et (f), R² représente un groupement 2,4,6-triisopropylphényle et n = 0.

3. Utilisation selon la revendication 1 ou 2 **caractérisée en ce que** le composé de formule I est le Nα-(2,4,6-triisopropylphénylsulfonyl)-3-guanidino-(D,L)-phénylalanine-4-éthoxycarbonyl-pipérazide, le Nα-(2,4,6-triisopropyl-phénylsulfonyl)-3-guanidino-(D,L)-phénylalanine-4-éthylaminocarbonyl-pipérazide, l'énantiomère L ou un sel pharmaceutiquement acceptable de l'un des composés.

4. Utilisation selon l'une des revendications 1 à 3 **caractérisée en ce que** les composés sont sous forme de sels d'acide physiologiquement acceptables, en particulier sous forme de chlorhydrates.

5. Utilisation selon l'une des revendications 1 à 4 pour la lutte contre les tumeurs.

6. Utilisation selon la revendication 5 pour la lutte contre le cancer du sein, le cancer du pancréas et la formation de métastases.

7. Utilisation selon l'une des revendications 1 à 4 pour la lutte contre pemphigus vulgaris.

8. Utilisation selon l'une des revendications 1 à 7 **caractérisée en ce que** les composés de formule I sont utilisés sous forme de conjugués avec d'autres substances pharmacologiquement efficaces.

9. Utilisation selon l'une des revendications 1 à 8 **caractérisée en ce que** les composés de formule I sont utilisés en combinaison avec d'autres substances pharmacologiquement efficaces.

10. Utilisation selon l'une des revendications 8 et 9 **caractérisée en ce que** les composés sont utilisés sous forme de conjugués avec des radiomarqueurs et/ou en combinaison avec des substances cytotoxiques.

11. Utilisation selon l'une des revendications 1 à 10 pour la production de médicaments pouvant être administrés par voie orale, topique, rectale ou parentérale.

12. Utilisation selon l'une des revendications 1 à 11 sous forme de comprimés, dragées, capsules, pellets, suppositoires, solutions ou systèmes transdermiques, comme des emplâtres.

13. Utilisation selon l'une des revendications 1-4 où le composé de formule (I) est un inhibiteur d'urokinase.

14. Composés de formule (I) 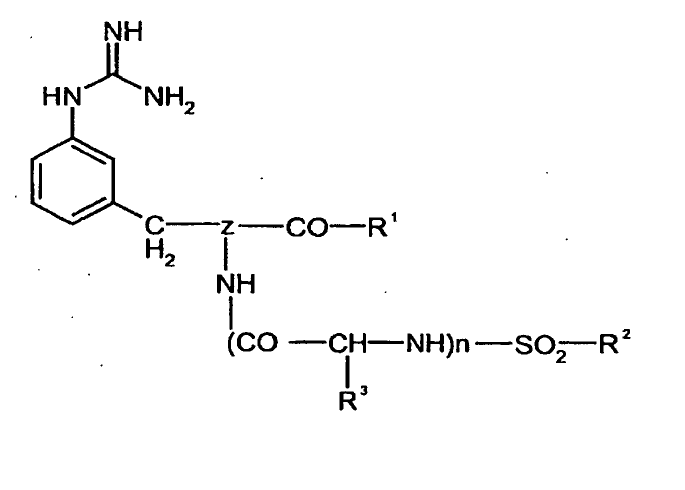 dans lesquels R¹ et n sont définis comme dans la revendication 2, R³ et Z sont définis comme dans la revendication 1 et R² est un groupement 2,4,6-triisopropylphényle.

15. Nα-(2,4,6-triisopropylphénylsulfonyl)-3-guanidino-(D,L)-phénylalanine-4-éthoxycarbonyl-pipérazide, Nα-(2,4,6-triisopropylphénylsulfonyl)-3-guanidino-(D,L)-phénylalanine-4-éthylaminocarbonyl-pipérazide, l'énantiomère L de ceux-ci ou un sel pharmaceutiquement acceptable de l'un des composés.

16. Composition pharmaceutique **caractérisée en ce qu'**elle contient comme principe actif un ou plusieurs composés selon la revendication 14 ou 15, éventuellement avec des vecteurs et/ou diluants pharmaceutiquement courants.
